# EUROPÄISCHE PATENTSCHRIFT

(11) **EP 1 799 275 B1**
(45) Veröffentlichungstag und Bekanntmachung des Hinweises auf die Patenterteilung: **24.10.2012**
(21) Anmeldenummer: 05791187.7
(22) Anmeldetag: 23.09.2005
(51) Int. Cl.: A61L 15/42, A61F 13/00, A61L 15/28, A61L 15/22

(54) **VERFAHREN ZUR HERSTELLUNG EINER WUNDAUFLAGE**
METHOD FOR THE PRODUCTION OF A WOUND PAD
PROCEDE DE PRODUCTION D'UN PANSEMENT

(30) Priorität: 27.09.2004 DE 102004047115
(43) Veröffentlichungstag der Anmeldung: 27.06.2007
(73) Patentinhaber: BSN Medical GmbH, 20253 Hamburg (DE)
(72) Erfinder: KULICKE, Werner, Michael, 22523 Hamburg (DE); WILHELMS, Tim, Axel, 56068 Koblenz (DE)
(74) Vertreter: UEXKÜLL & STOLBERG
(86) Internationale Anmeldenummer: PCT/DE2005/001686
(87) Internationale Veröffentlichungsnummer: WO 2006/034688

(56) Entgegenhaltungen:
- EP-A- 0 371 736
- EP-A- 0 415 183
- EP-B- 0 927 053
- WO-A-96/13284
- DE-A1- 4 322 956
- DE-A1- 19 729 905

## Beschreibung

Die Erfindung betrifft ein Verfahren zur Herstellung einer Wundauflage mit einer permeablen Polymermatrix.

Wundauflagen spielen bei der Behandlung von Problemwunden, wie Brandwunden, eine Rolle. Eine Brandwunde entsteht durch lokale Einwirkung von thermischer Energie oder ionisierenden oder UV-Strahlen auf die Körperoberfläche. Bei schweren Verbrennungen ist die Haut in so tiefen Schichten geschädigt, dass eine Hauttransplantation erfolgen muss. Eine Behandlung des Spenderareals (Spalthautentnahmewunde) erfolgt bislang noch im Operationssaal, das heißt unmittelbar postoperativ, mit kalten und warmen Wickeln zur Blutstillung. Anschließend wird die Wunde mit Hilfe von Salbenbandagen versorgt. Hierdurch entstehen hohe Personalkosten, weil täglich die Wunde gereinigt und die Bandagen gewechselt werden müssen. Die verwendeten Salbenbandagen verkleben auch am Wundgrund, weshalb es beim Bandagenwechsel zu einer erneuten Traumatisierung des Gewebes kommt. Des Weiteren stellen die Salbenbandagen keine Barriere für pathogene Keime dar, die die Wunde daher von außen infizieren können. Hierdurch wird die Epithelisierung der Wunde zusätzlich behindert.

Eine weitere Problemwunde stellt die chronische Wunde dar. Hierbei handelt es sich um eine Wunde, die nach vier bis sechs Wochen keine Tendenz zur Heilung zeigt. Die Wunde kann auf vielfältigen Wegen entstehen. Chronische Wunden oder auch verzögert heilende Wunden weisen meist eine Infektion des Wundareals auf. Häufig vorkommende chronische Wunden sind zum Beispiel Dekubitus und Ulcus curis.

Die Unterschiedlichkeit einzelner Wunden stellt auch besondere Anforderungen an die Wundauflagen. So muss bei stark exudierenden Wunden, wie beispielsweise Brandwunden oder Spalthautentnahmewunden, die aus der Wunde austretende Flüssigkeit aufgenommen bzw. abgeleitet werden, wobei ein Flüssigkeitsfilm auf der Wunde verbleiben soll. Bei chronischen Wunden, d.h. solchen Wunden, die wenig exudieren, ist dagegen eine geringere Permeabilität der Wundauflage wünschenswert.

Aus DE 43 22 956 C2 ist eine Folie aus Chitosan zur Wundversiegelung bekannt, mit der Hautablederungen, Brand-, Schürf-, Quetsch- und Defektwunden auch in großen Ausdehnungen sowie insbesondere das chronische Ulcus curis venosum versiegelt werden sollen. Die dort beschriebene Chitosanfolie weist eine den Gasaustausch fördernde perforierte Oberfläche auf, wobei der Gasaustausch im Wesentlichen über nachträglich in die Folie eingestanzte Atmungsöffnungen ermöglicht wird. Wie sich nämlich als Problem herausgestellt hatte, konnte mit einer bis dahin bekannten Chitosanfolie insbesondere bei großflächigen Wundversiegelungen keine ausreichende Atmung der Wunde sichergestellt werden. Die aus DE 43 22 956 C2 bekannte Folie ist aber schwierig herzustellen, zumal die mechanisch hergestellten Poren eine bestimmte Größe nicht überschreiten dürfen und kleinere Poren nur schwer zu erzeugen sind. Außerdem wird durch die Perforation die Barrierefunktion der Membran zerstört, da die hierdurch entstehenden Kanäle sich durch die gesamte Membran erstrecken und für Mikroorganismen einen ungehinderten Zugang zur Wunde verschaffen.

Darüber hinaus ist aus EP 0 927 053 B1 ein fragmentiertes polymeres Hydrogel bekannt, das insbesondere zur Vermeidung von Gewebeadhäsionen eingesetzt wird. Dieses Hydrogel kann auch einen Weichmacheranteil von 0,1...30 Gew.-% enthalten. Das Hydrogel stellt jedoch keine geeignete Wundauflage dar, die einen Gasaustausch fördert. Im Übrigen verfügt das Hydrogel über keine Poren.

DE 199 48 12 0 C2 beschreibt ein Verfahren zur Herstellung einer biologisch verträglichen dreidimensionalen Matrix mit Chitosan als Material für die Matrix. Die Matrix weist Poren auf, die dadurch entstehen, dass man eine wässrige Chitosanlösung einfriert und das Wasser bei vermindertem Druck absublimiert. Die Poren dienen dazu, menschliche oder tierische Zellen in die Matrix einwachsen zu lassen. Eine solche Matrix gewährleistet jedoch aufgrund des fehlenden Weichmachers keinen ausreichenden Gasaustausch.

Darüber hinaus sind aus DE 197 29 905 A1 als Wundabdeckungen verwendbare Trägermaterialien bekannt, die Blockcopolymere und Weichmacher enthalten. Trägermaterialien dieser Art weisen auch eine bestimmte Luft- und Wasserdurchlässigkeit auf, die jedoch nicht einstellbar ist.

Eine Membran, die sich zur Verwendung als Wundverschluss eignet, ist aus DE 690 29 969 T2 bekannt. Ein hierfür verwendeter Weichmacher diente lediglich dazu, den Elastizitätsgrad einzustellen.

Schließlich beschreibt DE 39 28 858 A1 eine Hydrogel-Wundauflage mit bestimmten Wasserdampfdurchlässigkeiten. Angaben zur Einstellbarkeit der Durchlässigkeiten erwähnt auch diese Druckschrift nicht.

Poröse Membranen sind weiterhin in DE 100 50 870 A1 und US 5,993,661 beschrieben. Auch bei diesen Membranen sind Permeabilitäten nicht einstellbar.

Aufgabe der vorliegenden Erfindung ist es daher, ein Verfahren bereitzustellen, mit dem Wundauflagen für unterschiedliche therapeutische Anwendungsbereiche herstellbar sind.

Erfindungsgemäß erfolgt die Lösung der gestellten Aufgabe durch ein Verfahren zur Herstellung einer Wundauflage mit einer permeablen Polymermatrix gelöst, das dadurch gekennzeichnet ist, dass man als Polymer Chitosan einsetzt und eine Permeabilität der Wundauflage vorgibt und in Abhängigkeit von der gewählten Permeabilität der Wundauflage einen Weichmacher einsetzt, bei dem es sich um Glycerin, einen Zitronensäureester, Glyceroltriacetat, 1,2-Propylenglykol oder Polyethylenglykol mit niedriger Molmasse von bis zu 400 g/mol handelt, wobei der Weichmacher gelöst wird und die Konzentration des Weichmachers in der Lösung entsprechend einer für die jeweilige Chitosanmatrix und den jeweiligen Weichmacher ermittelten Korrelation zwischen Weichmacherkonzentration und Permeabilität eingestellt wird, wobei man a) den Weichmacher zu einer Lösung eines Chitosans gibt und die mit dem Weichmacher versetzte Chitosanlösung anschließend zur Wundauflage weiterverarbeitet, oder b) eine Chitosanmembran herstellt und diese Chitosanmembran anschließend in die Lösung mit dem Weichmacher überführt.

Mit Hilfe dieses Verfahrens ist es erstmalig möglich, vor allem die Vorteile des Chitosans bei der Wundheilung, einschließlich der Hämostase, umfangreich zu nutzen. Da unterschiedliche Wundarten auch verschiedene Anforderungen an die Permeabilität von Wundauflagen stellen, war die Anwendung des Chitosans bislang sehr begrenzt.

Bei der Herstellung der Wundauflage kann man den Weichmacher zu einer Chitosanlösung geben und die mit dem Weichmacher versetzte Chitosanlösung anschließend zur Wundauflage weiterverarbeiten, in bevorzugter Weise zu einer Chitosanmembran in Form einer Folie. Alternativ hierzu besteht die Möglichkeit, dass man eine Chitosanmembran herstellt und diese Chitosanmembran anschließend in die Lösung mit dem Weichmacher überführt. In beiden Fällen führt der Weichmacher zu einer Erhöhung der Permeabilität der Wundauflage.

Mit Chitosan lassen sich vor allem stabile und zugleich flexible Wundauflagen herstellen. Bewährt hat sich hierbei die Verwendung von Chitosan mit einer gewichtsmittleren Molmasse von mindestens 100.000 g/mol. Chitosan ist grundsätzlich bekannt. Es handelt sich hierbei um mehr oder weniger deacetyliertes Chitin. In bevorzugter Weise wird ein Chitosansalz eingesetzt, das durch Entwässern einer sauren Lösung von Chitosan erhältlich ist.

Erfindungsgemäß handelt es sich bei dem Weichmacher um Glycerin, einen Zitronensäureester, Glyceroltriacetat, 1,2-Propandiol oder um 1,2-Propylenglykol. Ebenso lässt sich Polyethylenglykol (PEG) mit niedriger Molmasse von bis zu 400 g/mol verwenden. Bei PEG mit höheren Molmassen verringert sich die Elastizität und Flexibilität der Membran.

Überraschenderweise hat sich herausgestellt, dass mit zunehmender Konzentration des Weichmachers die Wasserdampfdurchlässigkeit der Wundauflage erhöht werden kann. Eine Erhöhung der Konzentration des Weichmachers in der Chitosanlösung bis auf 3,0 Gew.-% ergibt eine Membran, bei der das Chitosan und der Weichmacher in nahezu gleichen Mengen vorliegen. Nachfolgend wird dies am Beispiel Chitosan/Glycerin verdeutlicht, wobei auf die weiter unten angegebenen Ausführungsbeispiele zur Herstellung einer Chitosanmembran verwiesen wird.

| Chitosanlösung | Chitosanmembran |
|---|---|
| 0,25 Gew.-% Glycerin | 8 Gew.-% Glycerin |
| 0,50 Gew.-% Glycerin | 16 Gew.-% Glycerin |
| 0,75 Gew.-% Glycerin | 25 Gew.-% Glycerin |
| 1,00 Gew.-% Glycerin | 33 Gew.-% Glycerin |
| 2,00 Gew.-% Glycerin | 66 Gew.-% Glycerin |
| 3,00 Gew.-% Glycerin | 100 Gew.-% Glycerin |

100 Gew.-% Glycerin in der Chitosanmembran bedeutet, dass gleiche Gewichtsverhältnisse von Glycerin und Chitosan in der Membran vorliegen. Es muss jedoch davon ausgegangen werden, dass beim Trocknungsprozess auch Glycerin verdampft und somit der Glyceringehalt in der Membran geringere Werte als die angegebenen aufweisen kann.

Ein weiterer Vorteil der Chitosanmembran besteht darin, dass die äußere Oberfläche der Chitosanmembran nach Kontakt mit der feuchten Wunde elastisch und trocken bleibt. Die der Wunde zugewandte Seite erhält durch partielle Adsorption des Wundsekretes eine gallertartige Konsistenz. Dies führt sowohl zu einer Wundreinigung als auch zu einer Anpassung der Chitosanmembran an die Unebenheiten einer Wundoberfläche. Das physiologisch wichtige Feuchtmilieu der Wunde wird hierdurch während der exudativen Heilungsphase beibehalten. Außerdem verklebt die Chitosanmembran am Wundrand, so dass eine zusätzliche Fixierung der Membran nicht notwendig ist.

Eine nach dem erfindungsgemäßen Verfahren hergestellte Wundauflage weist eine den Gasaustausch durch die Wundauflage fördernde und einen Weichmacher enthaltende Chitosanmatrix auf, wobei die Chitosanmatrix über eine Porenstruktur verfügen kann, die durch vorübergehende Einlagerungen in die Chitosanmatrix erhältlich ist. Eine solche Wundauflage hat insbesondere den Vorteil, dass die auszubildenden Poren größenmäßig gut definierbar sind. So können beispielsweise Makroporen durch Einlagerungen von Silikatpartikeln und Mikroporen durch Einlagerungen von Polyethylenglykol mit einer Molmasse von 1.500...100.000 g/mol ausgebildet werden. Silikatpartikel bzw. Polyethylenglykol werden anschließend wieder aus der Chitosanmatrix herausgelöst. Auf diese Weise kann eine Porengröße von beispielsweise 0,1...100 µm eingestellt werden. Darüber hinaus kann bei der Wundheilung die Oberflächenstruktur der Wundauflage eine Rolle spielen, so dass es auch hierfür vorteilhaft ist, eine bestimmte Porenstruktur vorzusehen.

Insbesondere handelt es sich bei der Wundauflage um eine Chitosanmembran. Eine solche Membran kann unterschiedlich dick sein, wobei, je nach Anwendungsgebiet, 20...500 µm dicke Membranen verwendet werden.

Sollten an die Chitosanmembran besondere Anforderungen hinsichtlich ihrer Festigkeit gestellt sein, kann auch eine Vernetzung mittels Epichlorhydrin oder Dialdehyden wie Glutaraldehyd oder Glyoxal vorgenommen werden.

Andererseits kann die Wundauflage auch zwei- oder mehrschichtig aufgebaut sein, wobei die Polymermatrix mit einem Trägermaterial versehen ist, das aus synthetischen oder natürlichen Fasern bestehen kann. Vorzugsweise besteht das Trägermaterial aus Cellulose, Viskose, Polyamiden, Polyurethan, Polyester, Wolle oder Baumwolle. Das Trägermaterial sorgt für eine höhere mechanische Belastbarkeit der Wundauflage.

### Herstellungsbeispiele

### 1. Säurehaltige Chitosanmembran

Chitosan wird in einer verdünnten organischen oder anorganischen Säure gelöst, z.B. in Zitronensäure, Weinsäure, Ameisensäure, Salzsäure, Phosphorsäure, und vorzugsweise in Milchsäure oder Essigsäure. Zu dieser Lösung wird Weichmacher wie Glycerin in einer Konzentration von 0,01...3 % zugegeben. Diese Lösung wird auf eine Glasplatte oder Kunststoffplatte, insbesondere Plexiglas oder Polycarbonat, mit einer Schichtdicke von 1...10 mm aufgetragen. Diese so mit der Chitosanlösung beschichtete Platte wird bei einer Temperatur von 20...80 °C mehrere Stunden getrocknet. Die Membran kann anschließend leicht von der Syntheseplatte entfernt werden. Hinsichtlich einer technischen Herstellung können auch Polyesterfolien als Träger Verwendung finden. Es kann mit einer Wickeltechnik oder Abrolltechnik gearbeitet werden.

### 2. Neutrale Chitosanmembran

Die Herstellung erfolgt wie unter 1. beschrieben, wobei die Membran anschließend in einer alkalischen wässrigen Lösung neutralisiert wird. Für die Neutralisation kann Natronlauge oder Kalilauge verwendet werden. Die neutralisierte Chitosanmembran wird danach für einige Minuten in eine 10...80 %ige Glycerinlösung oder in eine 5...80 %ige PEG-Lösung gegeben. Schließlich wird die Membran bei einer Temperatur von 20...80°C mehrere Stunden getrocknet.

### 3. Saure, vernetze Chitosanmembran

Zu der Chitosanlösung wird ein Vernetzer wie Glyoxal oder Glutaraldehyd in einem Verhältnis bezogen auf eine Grundeinheit des Polymermonomers von 1/10...1/200 gegeben. Im Übrigen wird wie unter 1. beschrieben verfahren.

### 4. Neutrale, vernetze Chitosanmembranen

In Ergänzung zur Herstellung gemäß 3. wird die Membran anschließend in einer alkalischen, wässrigen Lösung neutralisiert. Danach erfolgt die erneute Weichmacherzugabe und Trocknung wie unter 2. beschrieben.

### 5. Poröse Chitosanmembran

In eine Chitosanlösung werden Silikatpartikel mit einer Größe von 5...60 µm in einer Konzentration von 1...20 % gegeben. Nach Herstellung und Trocknung der Membran werden die Silikatpartikel durch Zugabe von Natronlauge herausgelöst. Hierzu wird die Chitosanmembran für 1 bis 3 Stunden bei 50...100 °C in eine 5...20 %ige Natronlauge gegeben. Die so neutralisierte und poröse Chitosanmembran wird anschließend in eine 10...80 %ige Glycerinlösung oder in eine 5...80 % PEG-Lösung gegeben und danach erneut getrocknet.

### 6. Poröse Chitosanmembran mit Mikroporen

In eine Chitosanlösung wird 1...20 % PEG mit einer Molmasse von 1.500...100.000 g/mol gegeben. Nach Herstellung und Trocknung der Membran wird das PEG in einer verdünnten alkalischen Lösung herausgelöst. Hierzu wird die Chitosanmembran für 1 bis 24 Stunden bei 20... 60 °C in eine 0,1...3 % Natronlauge gegeben. Die so neutralisierte und poröse Chitosanmembran wird anschließend für einige Minuten in eine 10...80 % Glycerinlösung oder in eine 5...80 % PEG-Lösung gegeben. Die Membran wird anschließend bei einer Temperatur von 20...80 °C mehrere Stunden getrocknet.

### 7. Vernetzte Chitosanmembran

Die Herstellung erfolgt wie unter 1. beschrieben. Die Membran wird anschließend in einer alkalischen wässrigen Lösung neutralisiert und vernetzt. Hierzu wird die Chitosanmembran in eine 1...5 % Natronlauge, die zusätzlich Epichlorhydrin als Vernetzer enthält, gegeben. Das Epichlorhydrin wird in einer Konzentration von 0,000001...0,1 mol zugegeben. Die Chitosanmembran wird für 1 bis 5 Stunden bei 20...80 °C in dieser Lösung belassen. Die so neutralisierte und vernetzte Chitosanmembran wird anschließend für einige Minuten in eine 10...80 % Glycerinlösung oder in eine 5... 80 % PEG-Lösung gegeben. Die Membran wird anschließend bei einer Temperatur von 20...80 °C mehrer Stunden getrocknet.

### Bestimmung der Wasserdampfpermeabilität von Chitosanmembranen

Die Wasserdampfpermeablität einer 70 µm dicken Chitosanmembran wurde wie folgt bestimmt:

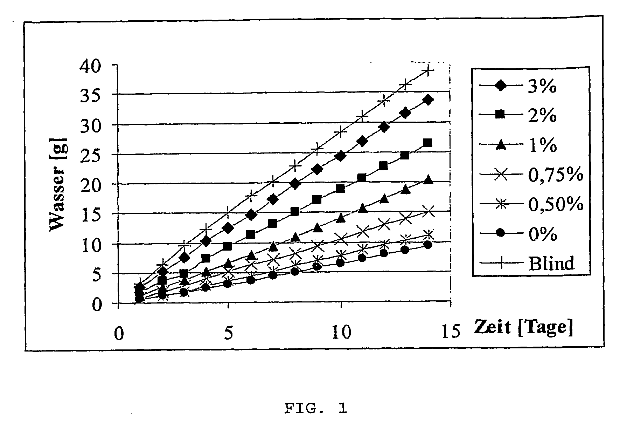

In eine 25 ml-Braunglasflasche wurden 5 g CaCl₂ (wasserfrei) eingewogen. Die Öffnung der Flasche wurde mit einer Chitosanmembran verschlossen. In eine zweite, unverschlossene Flasche wurde zur Kontrolle die gleiche Menge CaCl₂ gegeben. Beide Flaschen wurden in einen Exsikkator gestellt, der eine gesättigte NaCl-Lösung enthielt. Die relative Luftfeuchtigkeit innerhalb des Exsikkators wurde hierdurch auf 75 % eingestellt. Die Wasserdampfdurchlässigkeit der Chitosanmembran wurde dann täglich über einen Zeitraum von 14 Tagen durch Auswiegen der Proben ermittelt. Die Ergebnisse für Chitosanmembranen mit identischer Dicke und unterschiedlichen Glycerin-Gehalten der Membranen sind in der Abbildung dargestellt. Am 14. Versuchstag ergaben sich damit folgende Wasserdampfdurchlässigkeiten:

| Chitosanlösung | Wasserdampfdurchlässigkeit |
|---|---|
| 0,00 Gew.-% Glycerin | 24,1 % |
| 0,50 Gew.-% Glycerin | 28,9 % |
| 0,75 Gew.-% Glycerin | 38,8 % |
| 1,00 Gew.-% Glycerin | 52,6 % |
| 2,00 Gew.-% Glycerin | 68,0 % |
| 3,00 Gew.-% Glycerin | 86,9 % |

### Bestimmung der Porenstruktur

Poren der Chitosanmembranen lassen sich mittels der grundsätzlich bekannten Quecksilber-Porosimetrie (entwickelt von Rittner und Drake im Jahre 1945) sowie der Gas-Adsorption/- Desorption (BET, Langmuir) vermessen.

Nach dem Ausführungsbeispiel 5 hergestellte Membranen wiesen Makroporen mit einer Größe von zum Beispiel 60 µm auf, die der Größe der eingesetzten Silikatpartikel entsprachen. Die unter Verwendung von PEG hergestellten Mikroporen waren demgegenüber deutlich kleiner und hatten einen Porenradius von ca. 1...5 µm. Bei den makroporösen Membranen war die Porenzahl proportional zu der Zahl der eingesetzten Silikatpartikel.

Die Oberflächenstrukturen der mikro- oder makroporösen Membranen waren unterschiedlich rauh. Während die bei der Membranherstellung der Trägerplatte zugewandte Seite der Membran eine verhältnismäßig glatte Struktur aufwies, ließ die dem Träger abgewandte Seite der Membran eine rauhe, schwammartige Struktur erkennen.

## Patentansprüche

1. Verfahren zur Herstellung einer Wundauflage mit einer permeablen Polymermatrix, **dadurch gekennzeichnet, dass** man als Polymer Chitosan einsetzt und eine Permeabilität der Wundauflage vorgibt und in Abhängigkeit von der gewählten Permeabilität der Wundauflage einen Weichmacher einsetzt, bei dem es sich um Glycerin, einen Zitronensäureester, Glyceroltriacetat, 1,2-Propylenglykol oder Polyethylenglykol mit niedriger Molmasse von bis zu 400 g/mol handelt, wobei der Weichmacher gelöst wird und die Konzentration des Weichmachers in der Lösung entsprechend einer für die jeweilige Chitosanmatrix und den jeweiligen Weichmacher ermittelten Korrelation zwischen Weichmacherkonzentration und Permeabilität eingestellt wird, wobei man a) den Weichmacher zu einer Lösung eines Chitosans gibt und die mit dem Weichmacher versetzte Chitosanlösung anschließend zur Wundauflage weiterverarbeitet, oder b) eine Chitosanmembran herstellt und diese Chitosanmembran anschließend in die Lösung mit dem Weichmacher überführt.

2. Verfahren nach Anspruch 1, **dadurch gekennzeichnet, dass** es sich bei dem Chitosan um Chitosan mit einer gewichtsmittleren Molmasse von mindestens 100.000 g/mol handelt.

3. Verfahren nach Anspruch 2, **dadurch gekennzeichnet, dass** man anstelle des Chitosans ein Chitosansalz einsetzt, das durch Entwässern einer sauren Lösung von Chitosan erhältlich ist.

4. Verfahren nach Anspruch 1, **dadurch gekennzeichnet, dass** man den Weichmacher zu einer Chitosanlösung gibt und die mit dem Weichmacher versetzte Chitosanlösung anschließend zur Wundauflage weiterverarbeitet, wobei die Konzentration des Weichmachers in der Polymerlösung 0,01 ... 3,0 Gew.-% beträgt.

5. Verfahren nach Anspruch 4, **dadurch gekennzeichnet, dass** man die mit dem Weichmacher versetzte Chitosanlösung zu einer Chitosanmembran in Form einer Folie weiterverarbeitet.

6. Verfahren nach einem der Ansprüche 1 bis 5, **dadurch gekennzeichnet, dass** man die Porenstruktur der Chitosanmatrix einstellt.

7. Verfahren nach Anspruch 6, **dadurch gekennzeichnet, dass** man die Porenstruktur der Chitosanmatrix einstellt, indem der Chitosanlösung Partikel zusetzt und diese Partikel nach Weiterverarbeitung der Chitosanlösung zu einer Chitosanmembran aus der Chitosanmembran herauslöst.

8. Verfahren nach Anspruch 7, **dadurch gekennzeichnet, dass** es sich bei den Partikeln um Silikatpartikel handelt.

9. Verfahren nach Anspruch 6, **dadurch gekennzeichnet, dass** man die Porenstruktur der Chitosanmatrix einstellt, indem man der Chitosanlösung Polyethylenglykol zusetzt und das Polyethylenglykol nach Weiterverarbeitung der Chitosanlösung zu einer Chitosanmembran aus der Chitosanmembran herauslöst.

10. Verfahren nach Anspruch 9, **dadurch gekennzeichnet, dass** man Polyethylenglykol mit einer Molmasse von 1.500 ... 100.000 g/mol verwendet.

## Claims

1. A method for producing a wound dressing having a permeable polymer matrix, **characterized in that** the polymer used is chitosan and the permeability of the wound dressing is preselected and a plasticizer is used, depending on the selected permeability of the wound dressing, wherein the plasticizer is glycerol, a citric acid ester, glycerol triacetate, 1,2-propylene glycol or polyethylene glycol with a low molecular weight of up to 400 g/mol, wherein the plasticizer is dissolved and the concentration of the plasticizer in the solution is adjusted according to a correlation between the plasticizer concentration and the permeability ascertained for the particular chitosan matrix and the particular plasticizer, wherein a) the plasticizer is added to a solution of a chitosan and the chitosan solution mixed with the plasticizer is then processed further to produce the wound dressing, or b) a chitosan membrane is produced and this chitosan membrane is then transferred to the solution with the plasticizer.

2. The method according to claim 1, **characterized in that** the chitosan is chitosan with a weight-average molecular weight of at least 100,000 g/mol.

3. The method according to claim 2, **characterized in that** instead of the chitosan, a chitosan salt obtainable by dehydrating an acidic solution of chitosan is used.

4. The method according to claim 1, **characterized in that** the plasticizer is added to a chitosan solution and the chitosan solution mixed with the plasticizer is then processed further to produce the wound dressing, wherein the concentration of the plasticizer in the polymer solution is 0.01 to 3.0 percent by weight.

5. The method according to claim 4, **characterized in that** the chitosan solution mixed with the plasticizer is processed further to form a chitosan membrane in the form of a film.

6. The method according to any of claims 1 to 5, **characterized in that** the pore structure of the chitosan matrix is adjusted.

7. The method according to claim 6, **characterized in that** the pore structure of the chitosan matrix is adjusted by way of adding particles to the chitosan solution and then after further processing the chitosan solution to yield a chitosan membrane dissolving these particles out of the chitosan membrane.

8. The method according to claim 7, **characterized in that** the particles are silicate particles.

9. The method according to claim 6, **characterized in that** the pore structure of the chitosan matrix is adjusted by way of adding polyethylene glycol to the chitosan solution and then after further processing of the chitosan solution to form a chitosan membrane dissolving the polyethylene glycol out of the chitosan membrane.

10. The method according to claim 9, **characterized in that** polyethylene glycol with a molecular weight of 1500 to 100,000 g/mol is used.

## Revendications

1. Procédé de préparation d'un pansement comprenant une matrice polymère perméable, **caractérisé en ce que** l'on emploie, en tant que polymère, un chitosane et **en ce que** l'on prédétermine une perméabilité du pansement et, en fonction de cette perméabilité ainsi définie, on ajoute au pansement un plastifiant, lequel plastifiant consiste en glycérine, ester de l'acide citrique, triacétate de glycérol, 1,2-propylèneglycol ou polyéthylèneglycol présentant une masse moléculaire faible inférieure ou égale à 400 g/mole, procédé dans lequel le plastifiant est dissous dans la solution et la concentration du plastifiant dans la solution est réglée en fonction de la corrélation entre la concentration du plastifiant et la perméabilité satisfaisantes pour la matrice chitosane et le plastifiant employés, procédé dans lequel :
a) le plastifiant conduit à l'obtention d'une solution de chitosane et la solution de chitosane additionnée au plastifiant subit alors un traitement supplémentaire pour l'obtention d'un pansement, ou
b) on forme une membrane chitosane et ensuite, on ajoute cette membrane de chitosane à la solution contenant le plastifiant.

2. Procédé selon la revendication 1, **caractérisé en ce que**, en tant que chitosane, est employé un chitosane ayant une masse moléculaire moyenne au moins égale à 100 000 g/mole.

3. Procédé selon la revendication 2, **caractérisé en ce que**, au lieu du chitosane, on emploie un sel de chitosane, lequel est obtenu par élimination de l'eau d'une solution acide de chitosane.

4. Procédé selon la revendication 1, **caractérisé en ce que** l'on ajoute le plastifiant à une solution de chitosane, et **en ce que** ladite solution de chitosane additionnée de plastifiant subit alors un traitement supplémentaire pour l'obtention d'un pansement, procédé dans lequel la concentration du plastifiant dans la solution de polymère est comprise entre 0,01 et 3,0 % en poids.

5. Procédé selon la revendication 4, **caractérisé en ce que** la solution de chitosane additionnée de plastifiant comporte un traitement supplémentaire dans lequel la membrane de chitosane est mise sous forme d'une feuille.

6. Procédé selon l'une quelconque des revendications 1 à 5, **caractérisé en ce que** la matrice de chitosane comporte une structure poreuse.

7. Procédé selon la revendication 6, **caractérisé en ce que**, dans la structure poreuse de la matrice de chitosane, sont additionnées des particules et ces particules, après traitement supplémentaire, sont retirées par dissolution de la solution de chitosane pour former une membrane de chitosane.

8. Procédé selon la revendication 7, **caractérisé en ce que** les particules consistent en particules de silicate.

9. Procédé selon la revendication 6, **caractérisé en ce que** l'on confère à la matrice de chitosane une structure poreuse dans laquelle la solution de chitosane est additionnée de polyéthylèneglycol et le polyéthylèneglycol après traitement supplémentaire est retiré par dissolution de la solution de chitosane pour former une membrane de chitosane.

10. Procédé selon la revendication 9, **caractérisé en ce que** le polyéthylèneglycol comporte une masse moléculaire comprise entre 1500 et 100 000 g/mole.
